(19) Europäisches Patentamt
European Patent Office
Office européen des brevets

(11) EP 1 421 928 A2

(12) **DEMANDE DE BREVET EUROPEEN**

(43) Date de publication:
**26.05.2004 Bulletin 2004/22**

(51) Int Cl.[7]: **A61K 7/00**

(21) Numéro de dépôt: **03292383.1**

(22) Date de dépôt: **26.09.2003**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **26.09.2002 FR 0211949**
**20.12.2002 FR 0216437**
**21.05.2003 FR 0306121**

(71) Demandeur: **L'OREAL**
**75008 Paris (FR)**

(72) Inventeurs:
- **Toumi, Béatrice**
**91370 Verrieres le Buisson (FR)**
- **Lion, Bertrand**
**95270 Luzarches (FR)**
- **Leuridan, Frédéric**
**75005 Paris (FR)**

(74) Mandataire: **Boulard, Denis**
**L'OREAL - D.I.P.I.**
**25-29 Quai Aulagnier**
**92600 Asnières (FR)**

(54) **Composition de vernis à ongles comprenant un polymère séquencé**

(57) La présente invention a pour objet une composition de vernis à ongles comprenant, dans un milieu cosmétiquement acceptable comprenant un milieu solvant organique, au moins un polymère séquencé, ladite composition étant apte à former un film présentant un pouvoir amortissant tangente delta (tgδ) supérieur ou égal à 0,4 à une température de 30 °C et une fréquence de 20 Hz.

L'invention a encore pour objet l'utilisation d'une telle composition pour obtenir un film, déposé sur les ongles, brillant, de bonne tenue et résistant à l'usure.



Printed by Jouve, 75001 PARIS (FR)

## Description

La présente invention a pour objet un vernis à ongles comprenant un polymère séquencé. L'invention a également pour objet un procédé de maquillage ou de soin des ongles.

La composition de vernis à ongles peut être employée comme base pour vernis ou "base-coat" en terminologie anglo-saxonne, comme produit de maquillage des ongles, comme composition de finition, encore appelée "top-coat", à appliquer sur le produit de maquillage des ongles ou bien encore comme produit de soin cosmétique des ongles. Ces compositions peuvent s'appliquer sur les ongles d'êtres humains ou bien encore sur des faux ongles.

On connaît des compositions à appliquer par exemple sur l'ongle, de type vernis à ongles ou base de soin pour ongles en milieu solvant, comprenant de manière usuelle, au moins un polymère filmogène, éventuellement un agent plastifiant, des pigments, des agents rhéologiques et des solvants.

Actuellement, la nitrocellulose reste encore le filmogène principal le plus utilisé dans les vernis à ongles à solvants dans des formulations à brillance et tenue optimisées.
Les formulations comprenant des nitrocelluloses permettent d'obtenir des films avec un niveau de dureté et de brillance corrects mais qui manquent d'adhérence sur l'ongle.
On peut remédier à cet inconvénient, en ajoutant des plastifiants mais, dans ce cas, il faut utiliser des quantités très importantes de plastifiants et de co-résines, de l'ordre de celles de la nitrocellulose.
De plus, la présence de plastifiants dans ces formulations se traduit, après filmification et séchage, par une évolution des propriétés du film dans le temps, due à la fois à une lente évaporation des solvants résiduels contenus dans le film après séchage et à une perte potentielle d'une partie des plastifiants, notamment par évaporation, conduisant à un durcissement du film dans le temps et une mauvaise résistance à l'écaillage du film.

Les recherches effectuées pour remplacer la nitrocellulose par d'autres agents filmogènes tels que des polyacryliques et des polyuréthanes dans les vernis à ongles, comme par exemple les dispersions aqueuses de polyuréthanes décrits dans le document EP0648485, n'ont pas donné de résultats satisfaisant notamment en termes de tenue et de résistance aux facteurs externes tels que l'eau ou les détergents.

Le demandeur a découvert de façon surprenante qu'un vernis à ongles présentant un pouvoir amortissant tangente delta (tgδ) supérieur ou égal à 0,4 permet d'obtenir :

- une plastification des films sans recourir à l'adjonction de grandes quantités de plastifiants externes, tout en maintenant un bon niveau de dureté des films et
- une bonne résistance des vernis sur l'ongle aux chocs et/ou à l'écaillage et donc une amélioration de la tenue dans le temps des vernis sur l'ongle et/ou leur résistance à l'usure,
- tout en ayant un film de composition brillant.

De façon plus précise, l'invention a pour objet une composition de vernis à ongles comprenant, dans un milieu cosmétiquement acceptable comprenant un milieu solvant organique, au moins un polymère séquencé, ladite composition étant apte à former un film présentant un pouvoir amortissant tangente delta (tgδ) supérieur ou égal à 0,4 à une température de 30 °C et une fréquence de 20 Hz.

Par "milieu cosmétiquement acceptable", on entend au sens de l'invention un milieu non toxique et susceptible d'être appliqué sur la peau, les phanères ou les lèvres du visage d'êtres humains.
L'invention a aussi pour objet un procédé cosmétique de maquillage ou de soin non thérapeutique des ongles comprenant l'application sur les ongles d'au moins une couche de la composition de vernis à ongles telle que définie ci-dessus.

L'invention a encore pour objet l'utilisation d'une composition de vernis à ongles comprenant au moins un polymère séquencé, ladite composition étant apte à former un film présentant un pouvoir amortissant tangente delta (tgδ) supérieur ou égal à 0,4 à une température de 30°C et une fréquence de 20 Hz, pour obtenir un film, déposé sur les ongles, brillant, de bonne tenue et résistant à l'usure.

Ce polymère séquencé peut être formulé comme seul polymère filmogène ou en complément d'un polymère filmogène classique comme la nitrocellulose ou un dérivé de cellulose, sans avoir l'inconvénient dans ce dernier cas, de l'adjonction de grandes quantités de plastifiants.

La composition selon l'invention est apte à former un film caractérisé par un comportement viscoélastique particulier.

De façon générale, un matériau est dit viscoélastique quand, sous l'effet du cisaillement, il possède à la fois les caractéristiques d'un matériau purement élastique, c'est à dire capable de stocker de l'énergie et les caractéristiques d'un matériau purement visqueux, c'est à dire capable de dissiper de l'énergie et dont la réponse aux sollicitations est fonction du temps (réponse non instantanée).

Plus particulièrement, le film de la composition selon l'invention peut être caractérisé par son pouvoir amortissant tgδ, qui représente le rapport entre l'énergie dissipée et l'énergie transmise au sein du matériau.

La composition selon l'invention est apte à former un film ayant un pouvoir amortissant tgδ, supérieur ou égal à 0,4, notamment allant de 0,4 à 1.5, de préférence supérieur ou égal 0,5, notamment allant de 0,5 à 1.5, et mieux supérieur ou égal à 0,6, par exemple allant de 0,6 à 1, à une température de 30 °C et une fréquence de 20 Hz. Par ailleurs, la composition selon l'invention est de préférence apte à former un film ayant un module de conservation E' supérieur ou égal à 1 MPa, notamment allant de 1 MPa à 5000 MPa, de préférence supérieur ou égal à 5 MPa, notamment allant de 5 à 1000 MPa, et mieux supérieur ou égal à 10 MPa par exemple allant de 10 à 500 MPa à une température de 30 °C et une fréquence de 0,1 Hz.

Methodes de mesure des caractéristiques du film obtenu avec la composition

La mesure du pouvoir amortissant tgδ est effectuée par DMTA (Dynamical and Mechanical Temperature Analysis ou Analyse dynamique et mécanique en température). Pour mesurer le pouvoir amortissant tgδ du film de composition, on effectue des essais de viscoélasticimétrie avec un appareil DMTA de Polymer TA instruments (modèle DMA2980), sur un échantillon de film de composition. L'échantillon est préparé par coulage de la composition dans une matrice téflonnée puis séchage sur plaque thermostatée à 30 °C pendant 24 heures, sous des conditions d'humidité ambiante (typiquement 50% HR ± 15%). On obtient alors un film dans lequel on découpe (par exemple à l'emporte-pièce) les éprouvettes. Celles-ci ont typiquement une épaisseur d'environ 200 µm, une largeur de 5 à 10 mm et une longueur utile d'environ 10 à 15 mm, après 24h de séchage.
Les mesures sont effectuées à une température constante de 30°C.
L'échantillon est sollicité en traction et en petites déformations (on lui impose par exemple un déplacement sinusoïdal de ± 8 µm) lors d'un balayage en fréquence, la fréquence allant de 0,1 à 20 Hz. On travaille ainsi dans le domaine linéaire, sous de faibles niveaux de déformation.
Ces mesures permettent de déterminer le module complexe E* = E' + iE" du film de composition testé, E' étant le module de conservation et E" le module dit de perte.
De ces mesures, on déduit également le pouvoir amortissant : tgδ = E"/E'.

Déformation à la rupture

De préférence, la composition selon l'invention est apte à former un film ayant une déformation à la rupture $\varepsilon_r$ supérieure ou égale à 5 %, notamment allant de 5 à 500 %, de préférence supérieure ou égale 15%, notamment allant de 15 à 400% et/ou une énergie à rupture par unité de volume $W_r$ supérieure ou égale à 0,2 J/cm$^3$, notamment allant de 0,2 à 100 J/cm$^3$, de préférence supérieure à 1 J/cm$^3$, notamment allant de 1 à 50 J/cm$^3$.
La déformation à la rupture et l'énergie à rupture par unité de volume sont déterminées par des essais de traction effectués sur un film de composition d'environ 200 µm d'épaisseur. Le film est obtenu par coulage de la composition sur une matrice téflonnée puis séchage sur une plaque thermostatée à 30 °C pendant 7 jours, dans les conditions d'humidité ambiante.
Pour effectuer ces essais, le film est découpé en éprouvettes haltères de longueur utile 33 ± 1 mm et de largeur utile 6 mm. La section (S) de l'éprouvette est alors définie comme : S = largeur x épaisseur (cm$^2$) ; cette section sera utilisée pour le calcul de la contrainte.
Les essais sont réalisés, par exemple, sur un appareil de traction commercialisé sous l'appellation Lloyd® LR5K. Les mesures sont réalisées à température ambiante (20 °C).
Les éprouvettes sont étirées à une vitesse de déplacement de 33 mm/min, correspondant à une vitesse de 100 % d'allongement par minute.
On impose donc une vitesse de déplacement et on mesure simultanément l'allongement ΔL de l'éprouvette et la force F nécessaire pour imposer cet allongement. C'est à partir de ces données ΔL et F que l'on détermine les paramètres contraintes σ et déformation ε.
Il est ainsi obtenu une courbe contrainte $\sigma = (F/S)$ en fonction de la déformation $\varepsilon = (\Delta L/Lo) \times 100$, l'essai étant conduit jusqu'à rupture de l'éprouvette, $L_0$ étant la longueur initiale de l'éprouvette.
La déformation à la rupture $\varepsilon_r$ est la déformation maximale de l'échantillon avant le point de rupture (en %).
L'énergie à rupture par unité de volume Wr en J/cm$^3$ est définie comme la surface sous cette courbe contrainte/déformation telle que :

$$W_r = \int_0^{\varepsilon_r} \sigma.\varepsilon.d\varepsilon$$

1 ) Polymère séquencé

Le polymère séquencé de la composition selon l'invention est avantageusement un polymère éthylénique séquencé linéaire filmogène

Par polymère "éthylénique" , on entend un polymère obtenu par polymérisation de monomères comprenant une insaturation éthylénique.

Par polymère "séquencé", on entend un polymère comprenant au moins 2 séquences distinctes, de préférence au moins 3 séquences distinctes.

Le polymère est un polymère à structure linéaire. Par opposition, un polymère a structure non linéaire est, par exemple, un polymère à structure ramifiée, en étoile, greffée, ou autre.

Par polymère "filmogène", on entend un polymère apte à former à lui seul ou en présence d'un agent auxiliaire de filmification, un film continu et adhérent sur un support, notamment sur les matières kératiniques.

Avantageusement, le polymère séquencé de la composition selon la présente invention comprend au moins une première séquence et au moins une deuxième séquence de températures de transition vitreuses (Tg) différentes, lesdites première et deuxième séquences étant reliées entre elles par un segment intermédiaire comprenant au moins un monomère constitutif de la première séquence et au moins un monomère constitutif de la deuxième séquence.

De préférence, la première séquence et au moins la deuxième séquence du polymère séquencé sont incompatibles l'une avec l'autre.

Par "séquences incompatibles l'une avec l'autre", on entend que le mélange formé du polymère correspondant à la première séquence et du polymère correspondant à la deuxième séquence, n'est pas miscible dans le solvant organique majoritaire en poids du milieu solvant organique de la composition , à température ambiante (25°C) et pression atmosphérique ($10^5$ Pa), pour une teneur du mélange de polymères supérieure ou égale à 5 % en poids, par rapport au poids total du mélange (polymères et solvant), étant entendu que :

i) lesdits polymères sont présents dans le mélange en une teneur telle que le rapport pondéral respectif va de 10/90 à 90/10, et que
ii) chacun des polymères correspondant au première et seconde séquences a une masse moléculaire moyenne (en poids ou en nombre) égale à celle du polymère séquencé +/- 15%.

Dans le cas où le milieu solvant organique comprend un mélange de solvants organiques, dans l'hypothèse de deux ou plusieurs solvants présents en proportions massiques identiques, ledit mélange de polymères est non miscible dans au moins l'un d'entre eux.

Bien entendu, dans le cas où le milieu solvant organique comprend un unique solvant, ce dernier est le solvant majoritaire.

Le segment intermédiaire est une séquence comprenant au moins un monomère constitutif de la première séquence et au moins un monomère constitutif de la deuxième séquence du polymère permet de "compatibiliser" ces séquences.

De façon préférentielle, le polymère séquencé ne comprend pas d'atomes de silicium dans son squelette. Par "squelette", on entend la chaîne principale du polymère, par opposition aux chaînes latérales pendantes.

De préférence, le polymère séquencé de la composition selon l'invention n'est pas hydrosoluble, c'est à dire que le polymère n'est pas soluble dans l'eau ou dans un mélange d'eau et de monoalcools inférieurs linéaires ou ramifiés ayant de 2 à 5 atomes de carbone comme l'éthanol, l'isopropanol ou le n-propanol, sans modification de pH, à une teneur en matière active d'au moins 1 % en poids, à température ambiante (25°C).

De préférence, le polymère selon l'invention n'est pas un élastomère.

Par "polymère non élastomère", on entend un polymère qui, lorsqu'il est soumis à une contrainte visant à l'étirer (par exemple de 30% relativement à sa longueur initiale), ne revient pas à une longueur sensiblement identique à sa longueur initiale lorsque cesse la contrainte.

De manière plus spécifique, par "polymère non élastomére" on désigne un polymère ayant une recouvrance instantanée $R_i$ < à 50% et une recouvrance retardée $R_{2h}$ < 70% après avoir subi un allongement de 30%. De préférence, $R_i$ est < à 30 %, et $R_{2h}$ < 50.

Plus précisément, le caractère non élastomérique du polymère est déterminé selon le protocole suivant :

On prépare un film de polymère par coulage d'une solution du polymère dans une matrice téflonnée puis séchage pendant 7 jours dans une ambiance contrôlée à 23±5°C et 50±10% d'humidité relative.
On obtient alors un film d'environ 100 µm d'épaisseur dans lequel sont découpées des éprouvettes rectangulaires (par exemple à l'emporte-pièce) d'une largeur de 15 mm et d'une longueur de 80 mm.

On impose à cet échantillon une sollicitation de traction à l'aide d'un appareil commercialisé sous la référence

Zwick, dans les mêmes conditions de température et d'humidité que pour le séchage.

Les éprouvettes sont étirées à une vitesse de 50 mm/min et la distance entre les mors est de 50 mm, ce qui correspond à la longueur initiale ($l_0$) de l'éprouvette.

On détermine la recouvrance instantanée Ri de la manière suivante :

- on étire l'éprouvette de 30 % ($\varepsilon_{max}$) c'est-à-dire environ 0,3 fois sa longueur initiale ($l_0$)
- on relâche la contrainte en imposant une vitesse de retour égale à la vitesse de traction, soit 50 mm/min et on mesure l'allongement résiduel de l'éprouvette en pourcentage, après retour à contrainte charge nulle ($\varepsilon_i$).

La recouvrance instantanée en % ($R_i$) est donnée par la formule ci-après:

$$R_i = (\varepsilon_{max} - \varepsilon_i)/ \varepsilon_{max}) \times 100$$

Pour déterminer la recouvrance retardée, on mesure après 2 heures le taux l'd'allongement résiduel de l'éprouvette en pourcentage ($\varepsilon_{2h}$), 2 heures après retour à la contrainte charge nulle.

La recouvrance après 2hretardée en % ($R_{2h}$) est donnée par la formule ci-après:

$$R_{2h}= (\varepsilon_{max} - \varepsilon_{2h})/ \varepsilon_{max}) \times 100$$

A titre purement indicatif, unLe polymère selon un mode de réalisation de l'invention possède de préférence une recouvrance instantanée $R_i$ de 10% et une recouvrance retardée $R_{2h}$ de 30%.

Le polymère selon l'invention comprend au moins une première séquence (ou bloc) et au moins une deuxième séquence (ou bloc) ayant des températures de transition vitreuse (Tg) différentes, lesdites première et deuxième séquences étant reliées entre elles par un segment intermédiaire comprenant au moins un monomère constitutif de la première séquence et au moins un monomère constitutif de la deuxième séquence.

On précise que dans ce qui précède et ce qui suit les termes "première" et "deuxième" séquences ne conditionnent nullement l'ordre desdites séquences (ou blocs) dans la structure du polymère.

Avantageusement, le polymère séquencé de la composition selon l'invention a un indice de polydispersité 1 supérieur à 2, par exemple allant de 2 à 9, de préférence supérieur ou égal à 2,5, par exemple allant de 2,5 à 8, et mieux supérieur ou égal à 2,8 et notamment, allant de 2,8 à 6.

L'indice de polydispersité I du polymère est égal au rapport de la masse moyenne en poids Mw sur la masse moyenne en nombre Mn.

On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique).

La masse moyenne en poids (Mw) du polymère selon l'invention est de préférence inférieure ou égale à 300 000, elle va par exemple de 35 000 à 200 000, et mieux de 45 000 à 150 000.

La masse moyenne en nombre (Mn) du polymère selon l'invention est de préférence inférieure ou égale à 70 000, elle va par exemple de 10 000 à 60 000, et mieux de 12 000 à 50 000.

Chaque séquence ou bloc du polymère séquencé de la composition selon l'invention est issue d'un type de monomère ou de plusieurs types de monomères différents. Cela signifie que chaque séquence peut être constituée d'un homopolymère ou d'un copolymère ; ce copolymère constituant la séquence pouvant être à son tour statistique ou alterné.

Avantageusement, le segment intermédiaire comprenant au moins un monomère constitutif de la première séquence et au moins un monomère constitutif de la deuxième séquence du polymère est un polymère statistique.

De préférence, la séquence intermédiaire est issue essentiellement de monomères constitutifs de la première séquence et de la deuxième séquence.

Par "essentiellement", on entend au moins à 85%, de préférence au moins à 90%, mieux à 95% et encore mieux à 100%.

Avantageusement, la séquence intermédiaire du polymère séquencé a une température de transition vitreuse Tg comprise entre les températures de transition vitreuse des première et deuxième séquences.

Les première et deuxième séquences du polymère séquencé de la composition ont des températures de transition vitreuse différentes.

Les températures de transition vitreuse indiquées des première et deuxième séquences du polymère séquencé peuvent être des Tg théoriques déterminées à partir des Tg théoriques des monomères constitutifs de chacune des séquences, que l'on peut trouver dans un manuel de référence tel que le Polymer Handbook, 3rd ed, 1989, John

Wiley, selon la relation suivante, dite Loi de Fox :

$$1/Tg = \sum_i (\varpi_i / Tg_i) ,$$

$\omega_i$ étant la fraction massique du monomère i dans la séquence considerée et $Tg_i$ étant la température de transition vitreuse de l'homopolymère du monomère i.

Sauf indication contraire, les Tg indiquées pour les première et deuxième séquences du polymère séquencé dans la présente demande sont des Tg théoriques.

L'écart entre les températures de transition vitreuse des première et deuxième séquences du polymère séquencé est généralement supérieur à 10°C, de préférence supérieur à 20°C, et mieux supérieur à 30°C.

En particulier, la première séquence du polymère séquencé peut être choisie parmi :

- a) une séquence ayant une Tg supérieure ou égale à 40°C,
- b) une séquence ayant une Tg inférieure ou égale à 20°C,
- c) une séquence ayant une Tg comprise entre 20 et 40°C, et la deuxième séquence choisie dans une catégorie a), b) ou c) différente de la première séquence.

On entend désigner dans la présente invention, par l'expression :

« compris entre ... et ... », un intervalle de valeurs dont les bornes mentionnées sont exclues, et
« de ... à ... » et « allant de ... à ... », un intervalle de valeurs dont les bornes sont inclues.

<u>a) Séquence ayant une Tg supérieure ou égale à 40°C</u>

La séquence a yant une Tg supérieure ou égale à 40°C du polymère séquencé a p ar exemple une Tg allant de 40 à 150°C, de préférence supérieure ou égale à 50°C, allant par exemple de 50°C à 120 °C, et mieux supérieure ou égale à 60°C, allant par exemple de 60°C à 120°C .

La séquence ayant une Tg supérieure ou égale à 40°C peut être un homopolymère ou un copolymère.

Dans le cas où cette séquence est un homopolymère, elle est issue de monomères, qui sont tel(s) que les homopolymères préparés à partir de ces monomères ont des températures de transition vitreuse supérieures ou égales à 40°C. Cette première séquence peut être un homopolymère, constitué par un seul type de monomère (dont la Tg de l'homopolymère correspondant est supérieure ou égale à 40°C).

Dans le cas où la première séquence est un copolymère, elle peut être issue en totalité ou en partie de un ou de plusieurs monomères, dont la nature et la concentration sont choisies de façon que la Tg du copolymère résultant soit supérieure ou égale à 40°C. Le copolymère peut par exemple comprendre :

- des monomères qui sont tel(s) que les homopolymères préparés à partir de ces monomères ont des Tg supérieures ou égales à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C, et
- des monomères qui sont tel(s) que les homopolymères préparés à partir de ces monomères ont des Tg inférieures à 40°C, choisis parmi les monomères ayant une Tg comprise entre 20 à 40°C et/ou les monomères ayant une Tg inférieure ou égale à 20°C, par exemple une Tg allant de -100 à 20°C, de préférence inférieure à 15°C, notamment allant de - 80°C à 15°C et mieux inférieur à 10°C, par exemple allant de -50°C à 0°C à, tels que décrits plus loin, .

Les monomères dont les homopolymères ont une température de transition vitreuse supérieure ou égale à 40°C sont, de préférence, choisis parmi les monomères suivants, appelés aussi monomères principaux :

- les méthacrylates de formule $CH_2 = C(CH_3)\text{-}COOR_1$
  dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,
- les acrylates de formule $CH_2 = CH\text{-}COOR_2$
  dans laquelle $R_2$ représente un groupe cycloalkyle en $C_4$ à $C_{12}$ tel que l'acrylate d'isobornyle ou un groupe tertio butyle,
- les (méth)acrylamides de formule :

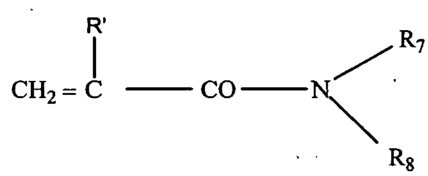

où $R_7$ et $R_8$ identiques ou différents représentent chacun un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl,
et R' désigne H ou méthyle. Comme exemple de monomères, on peut citer le
N-butylacrylamide, le N-t-butylacrylamide, le N-isopropylacrylamide, le N,N-diméthylacrylamide et le N,N-dibutylacrylamide ,

- et leurs mélanges.

Des monomères principaux particulièrement préférés sont le méthacrylate de méthyle, le (méth)acrylate d'isobutyle, le (méth)acrylate d'isobornyle et leurs mélanges.

b) Séquence ayant une Tg inférieure ou égale à 20°C

La séquence ayant une Tg inférieure ou égale à 20°C du polymère séquencé a par exemple une Tg allant de -100 à 20°C, de préférence inférieure ou égale à 15°C, notamment allant de - 80°C à 15°C et mieux inférieure ou égale à 10°C, par exemple allant de -50°C à 0°C.
La séquence ayant une Tg inférieure ou égale à 20°C peut être un homopolymère ou un copolymère.
Dans le cas où cette séquence est un homopolymère, elle est issue de monomères, qui sont tel(s) que les homopolymères préparés à partir de ces monomères ont des températures de transition vitreuse inférieures ou égales à 20°C. Cette deuxième séquence peut être un homopolymère, constitué par un seul type de monomère (dont la Tg de l'homopolymère correspondant est inférieure ou égale à 20°C).
Dans le cas où la séquence ayant une Tg inférieure ou égale à 20°C est un copolymère, elle peut être issue en totalité ou en partie de un ou de plusieurs monomères, dont la nature et la concentration sont choisis de façon que la Tg du copolymère résultant soit inférieure ou égale à 20°C. Elle peut par exemple comprendre

- un ou plusieurs monomères dont l'homopolymère correspondant a une Tg inférieure ou égale à 20°C, par exemple une Tg allant de -100°C à 20 °C, de préférence inférieure à 15°C, notamment allant de - 80°C à 15°C et mieux inférieur à 10°C, par exemple allant de -50°C à 0°C et
- un ou plusieurs monomères dont l'homopolymère correspondant a une Tg supérieure à 20°C, tels que les monomères ayant une Tg supérieure ou égale à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C et /ou les monomère ayant une Tg comprise entre 20 et 40°C, tels que décrits plus haut.

De préférence, la séquence ayant une Tg inférieure ou égale à 20°C est un homopolymère.
Les monomères dont l'homopolymère a une Tg inférieure ou égale à 20°C sont, de préférence, choisis parmi les monomères suivants, ou monomère principaux :

- les acrylates de formule $CH_2 = CHCOOR_3$,
  $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,

- les méthacrylates de formule $CH_2 = C(CH_3)\text{-}COOR_4$,
  $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;

- les esters de vinyle de formule $R_5\text{-CO-O-CH} = CH_2$
  où $R_5$ représente un groupe alkyle en $C_4$ à $C_{12}$ linéaire ou ramifié ;
  les éthers d'alcool vinylique et d'alcool en $C_4$ à $C_{12}$,

- les N-alkyl en $C_4$ à $C_{12}$ acrylamides, tels que le N-octylacrylamide,
- et leurs mélanges.

Les monomères principaux particulièrement préférés pour la séquence ayant une Tg inférieure ou égale à 20°C sont les acrylates d'alkyles dont la chaîne alkyle comprend de 1 à 10 atomes de carbone, à l'exception du groupe tertiobutyle, tels que l'acrylate de méthyle, l'acrylate d'isobutyle, l'acrylate d'éthyl-2 hexyle et leurs mélanges.

<u>c) Séquence ayant une Tg comprise entre 20 et 40°C</u>

La séquence qui a une Tg comprise entre 20 et 40°C du polymère séquencé peut être un homopolymère ou un copolymère.
Dans le cas où cette séquence est un homopolymère, elle est issue de monomères (ou monomère principaux), qui sont tel(s) que les homopolymères préparés à partir de ces monomères ont des températures de transition vitreuse comprises entre 20 et 40°C. Cette première séquence peut être un homopolymère, constitué par un seul type de monomère (dont la Tg de l'homopolymère correspondant va de 20°C à 40°C).
Les monomères dont l'homopolymère a une température de transition vitreuse comprise entre 20 et 40°C sont, de préférence, choisis parmi le méthacrylate de n-butyle, l'acrylate de cyclodécyle, l'acrylate de néopentyle, l'isodécylacrylamide et leurs mélanges.
Dans le cas où la séquence ayant une Tg comprise entre 20 et 40°C est un copolymère, elle est issue en totalité ou en partie de un ou de plusieurs monomères (ou monomère principaux), dont la nature et la concentration sont choisis de telle sorte que la Tg du copolymère résultant soit comprise entre 20 et 40°C.
Avantageusement, la séquence ayant une Tg comprise entre 20 et 40°C est un copolymère issue en totalité ou en partie :

- de monomères principaux dont l'homopolymère correspondant a une Tg supérieure ou égale à 40°C, par exemple une Tg allant de 40°C à 150°C, de préférence supérieure ou égale à 50°C, allant par exemple de 50 à 120°C, et mieux supérieure ou égale à 60°C, allant par exemple de 60°C à 120°C, tels que décrits plus haut, et/ou
- de monomères principaux dont l'homopolymère correspondant a une Tg inférieure ou égale à 20°C, par exemple une Tg allant de -100 à 20°C, de préférence inférieure ou égale à 15°C, notamment allant de -80°C à 15°C et mieux inférieure ou égale à 10°C, par exemple allant de -50°C à 0°C, tels que décrits plus haut,
  lesdits monomères étant choisis de telle sorte que la Tg du copolymère formant la première séquence est comprise entre 20 et 40°C.

De tels monomères principaux sont par exemple choisis parmi le méthacrylate de méthyle, l'acrylate et le méthacrylate d'isobornyle, l'acrylate de butyle, l'acrylate d'éthyl-2 hexyle et leurs mélanges.
De préférence, la proportion de la deuxième séquence ayant une Tg inférieure ou égale à 20°C va de 10 à 85% en poids du polymère, mieux de 20 à 70% et encore mieux de 20 à 50%.
Chacune des séquences du polymère séquencé peut néanmoins contenir en proportion minoritaire au moins un monomère constitutif de l'autre séquence.
Ainsi la première séquence du polymère séquencé peut contenir au moins un monomère constitutif de la deuxième séquence et inversement.
Chacune des première et/ou deuxième séquence du polymère séquencé, peu(ven)t comprendre, outre les monomères indiqués ci-dessus, un ou plusieurs autres monomères appelés monomères additionnels, différents des monomères principaux cités précédemment.
La nature et la quantité de ce ou ces monomères additionnels sont choisies de manière à ce que la séquence dans laquelle ils se trouvent ait la température de transition vitreuse désirée.
Ce monomère additionnel est par exemple choisi parmi :

a) les monomères hydrophiles tels que :

- les monomères à insaturation(s) éthylénique(s) comprenant au moins une fonction acide carboxylique ou sulfonique comme par exemple :

  l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,

- les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,
- les méthacrylates de formule $CH_2 = C(CH_3)\text{-}COOR_6$
dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,
- les méthacrylates de formule $CH_2 = C(CH_3)\text{-}COOR_9$,
$R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;
- les acrylates de formule $CH_2 = CHCOOR_8$,
$R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle,
ou $R_{10}$ représente un alkyle($C_1$-$C_{12}$)-O-POE (polyoxyéthylène) avec répétition du motif oxyéthylène de 5 à 30 fois, par exemple méthoxy-POE, ou $R_{10}$ représente un groupement polyoxyéthylèné comprenant de 5 à 30 motifs d'oxyde d'éthylène

b) les monomères à insaturation éthylénique comprenant un ou plusieurs atomes de silicium tels que le méthacryloxypropyl triméthoxy silane, le méthacryloxypropyl tris ( triméthylsiloxy ) silane,

- et leurs mélanges.

Des monomères additionnels particulièrement préférés sont l'acide acrylique, l'acide méthacrylique, le méthacrylate de trifluoroéthyle et leurs mélanges.

Selon un mode préféré de réalisation, le polymère séquencé de la composition selon l'invention est un polymère non siliconé, c'est à dire un polymère exempt d'atome de silicium.

Ce ou ces monomères additionnels représente(nt) généralement une quantité inférieure ou égale à 30% en poids, par exemple de 1 à 30% en poids, de préférence de 5 à 20% en poids et, de préférence encore, de 7 à 15% en poids du poids total des première et/ou deuxième séquences.

De préférence, chacune des première et deuxième séquences comprend au moins un monomère choisi parmi les esters d'acide (méth)acrylique, et éventuellement au moins un monomère choisi parmi l'acide (méth)acrylique, et leurs mélanges.
Avantageusement, chacune des première et deuxième séquences est issue en totalité d'au moins un monomère choisi parmi l'acide acrylique, les esters d'acide (méth)acrylique, et éventuellement d'au moins un monomère choisi parmi l'acide (méth)acrylique, et leurs mélanges

De préférence, le polymère séquencé de la composition selon l'invention est exempt de styrène. Par "polymère exempt de styrène", on entend un polymère contenant moins de 10 % en poids, par rapport au poids total du polymère, de préférence moins de 5 % en poids, mieux moins de 2 % en poids, mieux moins de 1 % en poids, voire ne contenant pas, de monomère styrénique comme le styrène, les dérivés de styrène tels que le méthylstyrène, le chlorostyrène ou le chlorométhylstyrène. de styrène ou de dérivés du styrène comme par exemple le méthylstyrène, le chlorostyrène ou le chlorométhylstyrène.

Le polymère séquencé de la composition selon l'invention peut être obtenu par polymérisation radicalaire en solution selon le procédé de préparation suivant :

- une partie du solvant de polymérisation est introduite dans un réacteur adapté et chauffée jusqu'à atteindre la température adéquate pour la polymérisation (typiquement entre 60 et 120°C),
- une fois cette température atteinte, les monomères constitutifs de la première séquence sont introduits en présence d'une partie de l'initiateur de polymérisation,
- au bout d'un temps T correspondant à un taux de conversion maximum de 90%, les monomères constitutifs de la deuxième séquence et l'autre partie de l'initiateur sont introduits,
- on laisse réagir le mélange pendant un temps T' ( allant de 3 à 6 h) au bout duquel le mélange est ramené à température ambiante,
- on obtient le polymère en solution dans le solvant de polymérisation.

Par solvant de polymérisation, on entend un solvant ou un mélange de solvants. Le solvant de polymérisation

peut être choisis notamment parmi l'acétate d'éthyle, l'acétate de butyle, les alcools tels que l'isopropanol, l'éthanol, les alcanes aliphatiques tels que l'isododécane et leurs mélanges. De préférence, le solvant de polymérisation est un mélange acétate de butyle et isopropanol ou l'isododécane.

Premier mode de réalisation

Selon un premier mode de réalisation, le polymère séquencé de la composition selon l'invention comprend au moins une (notamment une) première séquence ayant une Tg supérieure ou égale à 40°C, telle que décrite plus haut au a) et au moins une (notamment une) deuxième séquence ayant une Tg inférieure ou égale à 20°C, telle que décrite plus haut au b).

De préférence, la première séquence ayant une Tg supérieure ou égale à 40°C du polymère séquencé est un copolymère issu de monomères qui sont tels que l'homopolymère préparé à partir de ces monomères a u ne température d e transition vitreuse supérieure ou é gale à 40°C, tels que les monomère décrits plus haut.

Avantageusement, la deuxième séquence ayant une Tg inférieure ou égale à 20°C est un homopolymère issu de monomères qui sont tel(s) que l'homopolymère préparé à partir de ces monomères a une température de transition vitreuse inférieure ou égale à 20°C, tels que les monomères décrits plus haut.

De préférence, la proportion de la séquence ayant une Tg supérieure ou égale à 40°C du polymère séquencé va de 20 à 90% en poids du polymère, mieux de 30 à 80% et encore mieux de 50 à 70%.

De préférence, la proportion de la séquence ayant une Tg inférieure ou égale à 20°C va de 5 à 75% en poids du polymère, de préférence de 15 à 50% et mieux de 25 à 45%.

Ainsi, selon une première variante, le polymère séquencé de la composition selon l'invention peut comprendre :

- une première séquence de Tg supérieure ou égale à 40°C, par exemple ayant une Tg allant de 70 à 110°C, qui est un copolymère méthacrylate de méthyle / acide acrylique,
- une deuxième séquence de Tg inférieure ou égale à 20°C, par exemple allant de 0 à 20°C, qui est un homopolymère d'acrylate de méthyle et
- une séquence intermédiaire qui est un copolymère méthacrylate de méthyle/acide acrylique/acrylate de méthyle.

Selon une seconde variante, le polymère séquencé de la composition selon l'invention peut comprendre :

- une première séquence de Tg supérieure ou égale à 40°C, par exemple allant de 70 à 100°C, qui est un copolymère méthacrylate de méthyle/acide acrylique/méthacrylate de trifluoroéthyle,
- une deuxième séquence de Tg inférieure ou égale à 20°C, par exemple allant de 0 à 20°C, qui est un homopolymère d'acrylate de méthyle et
- une séquence intermédiaire qui est un copolymère statistique méthacrylate de méthyl/acide acrylique/acrylate de méthyle/méthacrylate de trifluoroéthyle.

Second mode de réalisation

Selon un second mode de réalisation, le polymère séquencé de la composition selon l'invention comprend une première séquence ayant une température de transition vitreuse (Tg) comprise entre 20 et 40°C, conforme aux séquences décrites au c) et une deuxième séquence ayant une température de transition vitreuse inférieure ou égale à 20°C, telle que décrite plus haut au b) ou une température de transition vitreuse supérieure ou égale à 40°C, telle que décrite au a) ci-dessus.

De préférence, la proportion de la première séquence ayant une Tg comprise entre 20 et 40°C va de 10 à 85% en poids du polymère, mieux de 30 à 80% et encore mieux de 50 à 70%.

Lorsque la deuxième séquence est une séquence ayant une Tg supérieure ou égale à 40°C, elle est de préférence présente en une proportion allant de 10 à 85% en poids du polymère, mieux de 20 à 70% et encore mieux de 30 à 70%.

Lorsque la deuxième séquence est une séquence ayant une Tg inférieure ou égale à 20°C, elle est de préférence présente en une proportion allant de 10 à 85% en poids du polymère, mieux de 20 à 70% et encore mieux de 20 à 50%.

De préférence, la première séquence ayant une Tg comprise entre 20 et 40°C du polymère séquencé est un copolymère issu de monomères qui sont tel(s) que l'homopolymère correspondant a une Tg supérieure ou égale à 40°C et de monomères qui sont tel(s) que l'homopolymère correspondant a une Tg inférieure ou égale à 20°C.

Avantageusement, la deuxième séquence ayant une Tg inférieure ou égale à 20°C ou ayant une Tg supérieure ou égale à 40°C est un homopolymère.

Avantageusement, le polymère séquencé de la composition selon l'invention comprend :

- une première séquence de Tg comprise entre 20 et 40°C, par exemple ayant une Tg de 25 à 39°C, qui est un copolymère comprenant au moins un monomère acrylate de méthyle, au moins un monomère méthacrylate de méthyle et au moins un monomère acide acrylique,
- une deuxième séquence de Tg supérieure ou égale à 40°C, par exemple allant de 85 à 125°C, qui est un homopolymère composé de monomères méthacrylate de méthyle et
- une séquence intermédiaire comprenant au moins un monomère acrylate de méthyle, méthacrylate de méthyle et
- une séquence intermédiaire comprenant au méthacrylate de méthyle, au moins un monomère acide acrylique et au moins un monomère acrylate de méthyle.

La composition selon l'invention comprend avantageusement de 0,1 à 60% en poids, de préférence de 0,5 à 50 % en poids, et de préférence encore de 1 à 40 % en poids dudit polymère séquencé par rapport au poids total de la composition.

<u>Milieu solvant organique</u>

Le milieu cosmétiquement acceptable de la composition cosmétique selon l'invention comprend un milieu solvant organique comprenant un solvant organique ou un mélange de solvants organiques.
Le solvant organique peut être choisi parmi :

- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la γ-butyrolactone ;
- les esters à chaîne courte (ayant de 3 à 8 atomes de carbone au total) tels que l'acétate d'éthyle, l'acétate de méthyle, l'acétate de propyle, l'acétate d'isopropyl, l'acétate de n-butyle, l'acétate d'isopentyle, l'acétate de méthoxypropyle, le lactate de butyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde ;
- les composés hétérocycliques tels que le tétrahydrofuranne ;
- le carbonate de propylène, le 3-éthoxypropionate d'éthyle ;
- leurs mélanges.

De préférence, le solvant est choisi parmi les esters à chaîne courte ayant de 3 à 8 atomes de carbone au total, tels que l'acétate d'éthyle, l'acétate de méthyle, l'acétate de propyle, l'acétate d'isopropyl, l'acétate de n-butyle, l'acétate d'isopentyle, l'acétate de méthoxypropyle, le lactate de butyle et leurs mélanges.
De préférence, le milieu solvant organique présente une polarité P allant de 0,422 à 0,725.
La polarité est définie en fonction des paramètres de solubilité selon l'espace de solubilité de Hansen suivant la relation suivante:

$$P = \sqrt{(\delta p^2 + \delta h^2)}/\delta t$$

- δh caractérisant les forces d'interactions spécifiques (type liaisons hydrogène, acide/base, donneur/accepteur, etc...) ;
- δp caractérisant les forces d'interactions de DEBYE entre dipôles permanents ainsi que les forces d'interactions de KEESOM entre dipôles induits et dipôles permanents.
- et $\delta t = \sqrt{(\delta p^2 + \delta h^2 + \delta d^2)}$, δd caractérisant les forces de dispersion de LONDON issues de la formation de dipôles induits lors des chocs moléculaires.

La définition et le calcul des paramètres de solubilité dans l'espace de solubilité tridimensionnel de HANSEN sont décrits dans l'article de C. M. HANSEN : "The three dimensionnal solubility parameters" J. Paint Technol. 39, 105 (1967) ;

Lorsque le milieu solvant comprend un mélange de solvants, la polarité est déterminée à partir des paramètres de solubilité du mélange qui sont eux mêmes déterminés à partir de ceux des composés pris séparément, selon les relations suivantes :

$$\delta dmel = \sum_i xi\ \delta di \quad ; \quad \delta \rho mel = \sum_i xi\ \delta pi \quad \text{et} \quad \delta hmel = \sum_i xi\ \delta hi$$

où xi représente la fraction volumique du composé i dans le mélange.

Comme solvant organique ayant une polarité allant de 0,422 à 0,725, on peut citer en particulier l'acétate de méthyle, l'acétate d'éthyle, l'acétate d'isopropyle, l'acétate de méthoxypropyle, le lactate de butyle, l'acétone, la méthyléthylcétone, le diacétone alcool, la γ-butyrolactone, le tétrahydrofuranne, le carbonate de propylène, le 3-éthoxypropionate d'éthyle, le diméthylsulfoxide, et leurs mélanges.

Le milieu solvant organique peut représenter de 10 à 95% en poids, par rapport au poids total de la composition, de préférence de 15% à 80% en poids, et mieux de 20 à 60% en poids.

Le milieu cosmétiquement acceptable de la composition selon l'invention peut éventuellement comprendre un milieu aqueux.

Polymère filmogène additionnel

La composition peut comprendre, outre le polymère séquencé de la composition selon l'invention, un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques.

Parmi les polymères filmogènes utilisables dans la composition de la présente invention, on peut citer les polymères synthétiques, de type radicalaire ou de type polycondensat, les polymères d'origine naturelle et leurs mélanges.

Le polymère filmogène peut être choisi en particulier parmi les polymères cellulosiques tels que la nitrocellulose, l'acétate de cellulose, l'acétobutyrate de cellulose, l'acétopropionate de cellulose, l'éthyl cellulose, ou bien encore les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines aryl-sulfonamide époxy.

Comme polymère filmogène, on peut notamment utiliser la nitrocellulose RS 1/8 sec ; RS ¼ sec. ; ½ sec. ; RS 5 sec. ; RS 15 sec. ; RS 35 sec. ; RS 75 sec.; RS 150 sec ; AS ¼ sec. ; AS ½ sec. ; SS ¼ sec. ; SS ½ sec. ; SS 5 sec., notamment commercialisée par la société HERCULES ; les résine toluène sulfonamide formaldéhyde "Ketjentflex MS80" de la société AKZO ou "Santolite MHP", "Santolite MS 80" de la société FACONNIER ou "RESIMPOL 80" de la société PAN AMERICANA, la résine alkyde "BECKOSOL ODE 230-70-E" de la société DAINIPPON, la résine acrylique "ACRYLOID B66" de la société ROHM & HAAS, la résine polyuréthane "TRIXENE PR 4127" de la société BAXENDEN.

Le polymère filmogène additionnel peut être présent dans la composition selon l'invention en une teneur allant de 0,1 % à 60 % en poids, par rapport au poids total de la composition, de préférence allant de 2 % à 40 % en poids, et mieux de 5 % à 25 % en poids.

Plastifiant

La composition peut comprendre, en outre, au moins un agent plastifiant. En particulier, on peut citer, seuls ou en mélange, les plastifiants usuels, tels que :

- les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther;
- les esters de glycérol,
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,

- des esters d'acides notamment carboxyliques, tels que des citrates, des phtalates, des adipates, des carbonates, des tartrates, des phosphates, des sébaçates,
- des dérivés oxyéthylénés tels que les huiles oxyéthylénées, notamment les huiles végétales telles que l'huile de ricin; leurs mélanges.

La quantité de plastifiant peut être choisie par l'homme du métier sur la base de ses connaissances générales, de manière à obtenir une composition ayant des propriétés cosmétiquement acceptables. De préférence, l'agent plastifiant est présent en une quantité inférieure à 20%, de préférence inférieur à 15% et mieux inférieure à 10%, encore mieux inférieure à 5% en poids par rapport au poids total de la composition. De préférence, la composition selon l'invention est exempte d'agent plastifiant

Matière colorante

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.
Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.
Parmi les pigments organiques, on peut citer le noir de carbone, les pigments de type D & C, et les laques à base de carmin de cochenille, de baryum, strontium, calcium, aluminium.
Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.
Les colorants hydrosolubles sont par exemple le jus de betterave, le bleu de méthylène.

La composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.
Les charges peuvent être minérales ou organiques de toute forme, plaquettaires, sphériques ou oblongues, quelle que soit la forme cristallographique ( par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon@), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads@ de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium
ou de lithium, le laurate de zinc, le myristate de magnésium.

<u>Autres Additifs</u>

La composition peut comprendre, en outre, d'autres ingrédients utilisés couramment dans les compositions cosmétiques. De tels ingrédients peuvent être choisis parmi les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants.

Bien entendu, l'homme du métier veillera à choisir ce ou ces éventuels composés complémentaires, et/ou

leur quantité, de manière telle que les propriétés avantageuses de la composition pour l'utilisation selon l'invention ne soient pas, ou substantiellement pas, altérées par l'adjonction envisagée.

Selon un autre aspect, l'invention a pour objet un produit de vernis à ongles comprenant : i) un récipient délimitant au moins un compartiment, ledit récipient étant fermé par un élément de fermeture et ii) une composition selon l'invention qui est disposée à l'intérieur dudit compartiment.

Le récipient peut être sous toute forme adéquate. Il peut être notamment sous forme d'une bouteille et peut être, au moins pour partie, en un matériau tel que le verre. Toutefois, des matériaux autres que le verre peuvent être utilisés comme les matériaux thermoplastiques tels que le PP ou le PE ou comme un métal.

L'élément de fermeture peut être couplé au compartiment par vissage en position fermée du récipient. Alternativement, le couplage entre l'élément de fermeture et le récipient peut se faire autrement que par vissage, notamment par encliquetage.

Le récipient est de préférence équipé d'un applicateur pouvant être sous forme d'un pinceau constitué d'au moins une touffe de poils. Alternativement, l'applicateur se présente sous forme autre qu'un pinceau, par exemple sous forme d'une spatule ou d'un embout en mousse.

Les exemple qui suivent illustrent de manière non limitative l'invention.

Dans les exemples qui suivent, les Tg indiquées pour les première et deuxième séquences sont des Tg théoriques calculées de la manière définie précédemment.

## Exemple 1 : Préparation d'un polymère de poly(méthacrylate de méthyle/acide acrylique/acrylate de méthyle)

100 g d'acétate de butyle sont introduits dans un réacteur de 1 litre, puis on augmente la température de façon à passer de la température ambiante (25°C) à 90°C en 1 heure.

On ajoute ensuite, à 90°C et en 1 heure, 180 g de méthacrylate de méthyle, 30 g d'acide acrylique, 40 g d'acétate de butyle, 70 g d'isopropanol et 1,8 g de 2.5- Bis(2-éthylhexanoylperoxy)-2.5-diméthylhexane (Trigonox® 141 d'Akzo Nobel).

Le mélange est maintenu 1 heure à 90°C.

On introduit ensuite au mélange précédent, toujours à 90°C et en 1 heure, 90 g d'acrylate de méthyle, 70 g d'acétate de butyle, 20 g d'isopropanol et 1,2 g de 2.5- Bis(2-éthylhexanoylperoxy)-2.5-diméthylhexane.

Le mélange est maintenu 3 heures à 90°C, puis dilué par 105 g d'acétate de butyle et 45 g d'isopropanol, puis l'ensemble est refroidi.

On obtient une solution à 40% de matière active en polymère dans le mélange acétate de butyle / isopropanol.

On obtient un polymère comprenant une première séquence ou bloc poly (méthacrylate de méthyle/acide acrylique) ayant une Tg de 100°C une deuxième séquence ou bloc polyacrylate de méthyle ayant une Tg de 10°C et une séquence intermédiaire qui est un polymère statistique méthacrylate de méthyle/acide acrylique/polyacrylate de méthyle.

Ce polymère présente une masse moyenne en poids de 52000 et une masse moyenne en nombre de 18000, soit un indice de polydispersité I de 2,89

Il présente un module de conservation E' égal à 90 MPa, à 30°C et 0,1Hz et une valeur de tgδ de 0,33 à 30°C et 20 Hz.

## Exemple 2 : Préparation d'un polymère de poly (méthacrylate de méthyle /acide acrylique/acrylate de méthyle)

100 g d'acétate de butyle sont introduits dans un réacteur de 1 litre, puis on augmente la température de façon à passer de la température ambiante (25°C) à 90°C en 1 heure.

On ajoute ensuite, à 90°C et en 1 heure, 150g de méthacrylate de méthyle, 30 g d'acide acrylique, 30 g d'acrylate de méthyle, 40 g d'acétate de butyle, 70 g d'isopropanol et 1,8 g de 2.5- Bis(2-éthylhexanoylperoxy)-2.5-diméthylhexane (Trigonox® 141 d'Akzo Nobel).

Le mélange est maintenu 1 heure à 90°C.

On introduit ensuite au mélange précédent, toujours à 90°C et en 1 heure, 90 g d'acrylate de méthyle, 70 g d'acétate de butyle, 20 g d'isopropanol et 1,2 g de 2.5- Bis(2-éthylhexanoylperoxy)-2.5-diméthylhexane.

Le mélange est maintenu 3 heures à 90°C, puis dilué par 105 g d'acétate de butyle et 45 g d'isopropanol, puis l'ensemble est refroidi.

On obtient une solution à 40% de matière active en polymère dans le mélange acétate de butyle / isopropanol.

On obtient un polymère comprenant une première séquence ou bloc poly (acide acrylique/acrylate de méthyle) ayant une Tg de 80°C, une deuxième séquence polyacrylate de méthyle ayant une Tg de 10°C et une séquence intermédiaire qui est un polymère statistique acide acrylique/acrylate de méthyle/polyacrylate de méthyle.

Ce polymère présente une masse moyenne en poids de 50 000 et une masse moyenne en nombre de 17 000, soit un indice de polydispersité I de 2,95.

Il présente un module de conservation E' égal à 12 MPa à 30°C et 0,1Hz et une valeur de tgδ de 0,54 à 30°C et 20 Hz.

**Exemple 3 : Préparation d'un polymère de poly(acide acrylique/acrylate de méthyle/acrylate de méthyle/méthacrylate de trifluoroéthyle)**

100 g d'acétate de butyle sont introduits dans un réacteur de 1 litre, puis on augmente la température de façon à passer de la température ambiante (25°C) à 90°C en 1 heure. On ajoute ensuite, à 90°C et en 1 heure, 120 g de méthacrylate de méthyle, 30 g d'acide acrylique, 60 g de méthacrylate de trifluoroéthyle, 40 g d'acétate de butyle, 70 g d'isopropanol et 1,8 g de 2.5- Bis(2-éthylhexanoylperoxy)-2.5-diméthylhexane (Trigonox® 141 d'Akzo Nobel).
Le mélange est maintenu 1 heure à 90°C.
On introduit ensuite au mélange précédent, toujours à 90°C et en 1 heure, 90 g d'acrylate de méthyle, 70 g d'acétate de butyle, 20 g d'isopropanol et 1,2 g de 2.5- Bis(2-éthylhexanoylperoxy)-2.5-diméthylhexane.
Le mélange est maintenu 3 heures à 90°C, puis dilué par 105 g d'acétate de butyle et 45 g d'isopropanol, puis l'ensemble est refroidi.
On obtient une solution à 40% de matière active en polymère dans le mélange acétate de butyle / isopropanol.

On obtient un polymère comprenant une première séquence ou bloc poly (acide acrylique/méthacrylate de méthyle/méthacrylate de trifluoroéthyle) ayant une Tg de 85°C, une deuxième séquence polyacrylate de méthyle ayant une Tg de 10°C et une séquence intermédiaire qui est un polymère statistique acide acrylique/acrylate de méthyle/ polyacrylate de méthyle/méthacrylate de trifluoroéthyle.

Ce polymère présente une masse moyenne en poids de 53 000 et une masse moyenne en nombre de 17 500, soit un indice de polydispersité I de 3,03.

Il présente un module de conservation E' égal à 3 MPa à 30°C et 0,1 Hz et une valeur de tgδ de 0.34 à 30°C et 20 Hz.

**Exemple 4 : Préparation d'un polymère de poly(méthacrylate de méthyle/acrylate de méthyle/acide acrylique)**

210 g d'acétate d'éthyle sont introduits dans un réacteur de 1 litre, puis on augmente la température de façon à passer de la température ambiante (25°C) à 78°C en 1 heure.
On ajoute ensuite, à 78°C et en 1 heure, 54 g de méthacrylate de méthyle, 21 g d'acide acrylique, 135 g d'acrylate de méthyle et 1,8 g de 2.5-Bis(2-éthylhexanoylperoxy)-2.5-diméthylhexane (Trigonox® 141 d'Akzo Nobel).
Le mélange est maintenu 1 heure à 90°C.
On introduit ensuite au mélange précédent, toujours à 78°C et en 1 heure, 90 g de méthacrylate de méthyle, 90 g d'acétate d'éthyle et 1,2 g de 2.5-Bis(2-éthylhexanoylperoxy)-2.5-diméthylhexane.
Le mélange est maintenu 3 heures à 78°C, puis dilué par 150 g d'acétate d'éthyle puis l'ensemble est refroidi.
On obtient une solution à 40% de matière active en polymère dans l'acétate d'éthyle.

Le polymère obtenu comprend une première séquence ou bloc poly(acrylate de méthyle/méthacrylate de méthyle/acide acrylique) ayant une Tg de 35°C une deuxième séquence ou bloc poly (méthacrylate de méthyle ) ayant une Tg de 100°C et une séquence intermédiaire qui est un polymère statistique méthacrylate de méthyle/acide acrylique/polyacrylate de méthyle.

Ce polymère présente une masse moyenne en poids de 141 000 et une masse moyenne en nombre de 50 000, soit un indice de polydispersité I de 2,82

**Exemple 5 : Préparation d'un polymère de poly(méthacrylate de méthyle/acrylate** de **méthyle/acide acrylique)**

100 g d'acétate de butyle sont introduits dans un réacteur de 1 litre, puis on augmente la température de façon à passer de la température ambiante (25°C) à 90°C en 1 heure.
On ajoute ensuite, à 90°C et en 1 heure, 50,4 g de méthacrylate de méthyle, 21 g d'acide acrylique, 138,6 g d'acrylate de méthyle, 40 g d'acétate de butyle, 70 g d'isopropanol et 1,8 g de 2.5-Bis(2-éthylhexanoylperoxy)-2.5-diméthylhexane (Trigonox® 141 d'Akzo Nobel).
Le mélange est maintenu 1 heure à 90°C.
On introduit ensuite au mélange précédent, toujours à 90°C et en 1 heure, 90 g de méthacrylate de méthyle, 70 g d'acétate de butyle, 20 g d'isopropanol et 1,2 g de 2.5-Bis(2-éthylhexanoylperoxy)-2.5-diméthylhexane.
Le mélange est maintenu 3 heures à 90°C, puis dilué par 105 g d'acétate de butyle et 45 g d'isopropanol, puis l'ensemble est refroidi.
On obtient une solution à 40% de matière active en polymère dans le mélange acétate de butyle / isopropanol.

Le polymère obtenu comprend une première séquence ou bloc poly(acrylate de méthyle/méthacrylate de méthyle/acide acrylique) ayant une Tg de 35°C une deuxième séquence ou bloc poly (méthacrylate de méthyle ) ayant une Tg de 100°C et une séquence intermédiaire qui est un polymère statistique méthacrylate de méthyle/acide acrylique/polyacrylate de méthyle.

**Exemple 6 : Vernis à ongles**

On a préparé un vernis à ongles ayant la composition suivante

| | |
|---|---|
| Polymère de l'exemple 1 | 23,8 g en MA |
| Acétate de Butyle | 24,99 g |
| Isopropanol | 10,71 g |
| Hexylène Glycol | 2,5 g |
| DC RED 7 Lake | 1 g |
| Hectorite modifiée par du chlorure de stéaryl di méthyl benzyl ammonium (Bentone® 27V d'Elementis) | 1,3 g |
| Acétate d'éthyle | qsp 100 g |

Après application sur les ongles, le film de vernis a été jugé comme présentant de très bonnes propriétés de tenue et de résistance aux chocs.

On peut préparer la composition suivante :

**Exemple 7 : Vernis à ongles**

| | |
|---|---|
| Polymère de l'exemple 4 | 23,8 g en MA |
| Acétate de Butyle | 24,99 g |
| Isopropanol | 10,71 g |
| DC RED 7 Lake | 1 g |
| Hectorite modifiée par du chlorure de stéaryl di méthyl benzyl ammonium (Bentone® 27V d'Elementis) | 1,3 g |
| Acétate d'éthyle | qsp 100 g |

**Revendications**

1. Composition de vernis à ongles comprenant, dans un milieu cosmétiquement acceptable comprenant un milieu solvant organique, au moins un polymère séquencé, ladite composition étant apte à former un film présentant un pouvoir amortissant tangente delta (tgδ) supérieur ou égal à 0,4 à une température de 30 °C et une fréquence de 20 Hz.

2. Composition selon la revendication précédente **caractérisée en ce que** le film de composition présente un pouvoir amortissant tangente delta tgδ supérieure ou égale à 0,5 à une température de 30 °C et une fréquence de 20 Hz.

3. Composition selon l'une des revendications précédentes **caractérisée en ce que** le film de composition présente un module de conservation E' supérieur ou égal à 1 MPa, de préférence supérieur ou égal à 5 MPa, et mieux supérieur ou égal à 10 MPa à une température de 30 °C et une fréquence de 0,1 Hz.

4. Composition selon l'une des revendications précédentes, **caractérisée en ce que** la composition est apte à former un film ayant une déformation à la rupture $\varepsilon_r$ supérieure ou égale à 5 %, notamment allant de 5 à 500 %, de préférence supérieure ou égale 15%, notamment allant de 15 à 400% et/ou une énergie à la rupture par unité de volume $W_r$ supérieure ou égale à 0,2 J/$cm^3$ à une température de 20°C.

5. Composition selon l'une des revendications précédentes, **caractérisée en ce que** le polymère séquencé comprend au moins une première séquence et au moins une deuxième séquence de températures de transition vitreuses (Tg) différentes, lesdites première et deuxième séquences étant reliées entre elles par un segment intermédiaire comprenant au moins un monomère constitutif de la première séquence et au moins un monomère constitutif de la deuxième séquence.

**6.** Composition selon l'une des revendicaitons précédentes, **caractérisée en ce que** les première et deuxième séquences sont incompatibles.

**7.** Composition selon la revendication précédente, **caractérisé** en ce q ue la première séquence du polymère séquencé est choisie parmi :

- a) une séquence ayant une Tg supérieure ou égale à 40°C,
- b) une séquence ayant une Tg inférieure ou égale à 20°C,
- c) une séquence ayant une Tg comprise entre 20 et 40°C, et

la deuxième séquence est choisie dans une catégorie a), b) ou c) différente de la première séquence.

**8.** Composition selon la revendications précédente, **caractérisée en ce que** la séquence ayant une Tg supérieure ou égale à 40°C du polymère séquencé est issue en totalité ou en partie de un ou plusieurs monomères, qui sont tels que l'homopolymère préparé à partir de ces monomères a une températures de transition vitreuse supérieure ou égale à 40°C.

**9.** Composition selon la revendication précédente, **caractérisée en ce que** les monomères dont l'homopolymère correspondant a une température de transition vitreuse supérieure ou égale à 40°C sont choisis parmi les monomères suivants :

- les méthacrylates de formule $CH_2 = C(CH_3)\text{-}COOR_1$
dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,
- les acrylates de formule $CH_2 = CH\text{-}COOR_2$
dans laquelle $R_2$ représente un groupe cycloalkyle en $C_4$ à $C_{12}$ tel que l'acrylate d'isobornyle ou un groupe tertio butyle,
- les (méth)acrylamides de formule :

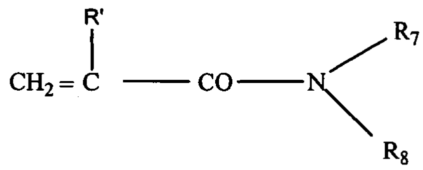

où $R_7$ et $R_8$ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl, et R' désigne H ou méthyle.
- et leurs mélanges.

**10.** Composition selon la revendication 8 ou 9, **caractérisée en ce que** les monomères dont l'homopolymère correspondant a une température de transition vitreuse supérieure ou égale à 40°C sont choisis parmi le méthacrylate de méthyle, le méthacrylate d'isobutyle, le (méth)acrylate d'isobornyle et leurs mélanges.

**11.** Composition selon la revendication 7, **caractérisée en ce que** la séquence ayant une Tg inférieure ou égale à 20°C du polymère séquencé est issue en totalité ou en partie de un ou plusieurs monomères, qui sont tel(s) que l'homopolymère préparé à partir de ces monomères a une température de transition vitreuse inférieure ou égale à 20°C.

**12.** Composition selon la revendication précédente, **caractérisée en ce que** les monomères dont l'homopolymère

correspondant a une température de transition vitreuse inférieure ou égale à 20°C sont choisis parmi les monomères suivants :

- les acrylates de formule $CH_2$ = $CHCOOR_3$, $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S ;
- les méthacrylates de formule $CH_2$ = $C(CH_3)$-$COOR_4$, $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule $R_5$-CO-O-CH = $CH_2$

où $R_5$ représente un groupe alkyle en $C_4$ à $C_{12}$ linéaire ou ramifié ;

- les éthers de vinyle et d'alkyle en $C_4$ à $C_{12}$, tels que l'éther de vinyle et de méthyle et l'éther de vinyle et d'éthyl ;
- les N-alkyl en $C_4$ à $C_{12}$ acrylamides, tels que le N-octylacrylamide,
- et leurs mélanges.

**13.** Composition selon la revendication précédente, **caractérisée en ce que** les monomères dont l'homopolymère correspondant a une température de transition vitreuse inférieure ou égale à 20°C sont choisis parmi les acrylates d'alkyle dont la chaîne alkyle comprend de 1 à 10 atomes de carbone, à l'exception du groupe tertiobutyle.

**14.** Composition selon la revendication 7, **caractérisée en ce que** la séquence ayant une Tg comprise entre 20 et 40°C est issue en totalité ou en partie de un ou de plusieurs monomères, qui sont tel(s) que l'homopolymère préparés à partir de ces monomères a une température de transition vitreuse comprise entre 20 et 40°C.

**15.** Composition selon la revendication précédente, **caractérisée en ce que** la séquence ayant une Tg comprise entre 20 et 40°C est issue en totalité ou en partie de monomères qui sont tel(s) que l'homopolymère correspondant a une Tg supérieure ou égale à 40°C et de monomères qui sont tel(s) que l'homopolymère correspondant a une Tg inférieure ou égale à 20°C.

**16.** Composition selon la revendication 14 ou 15, **caractérisée en ce que** la séquence ayant une Tg comprise entre 20 et 40°C est issue en totalité ou en partie de monomères choisis parmi le méthacrylate de méthyle, l'acrylate et le méthacrylate d'isobornyle, le méthacrylate de trifuoroéthyle, l'acrylate de butyle, l'acrylate d'éthyl-2 hexyle et leurs mélanges.

**17.** Composition selon l'une des revendications 1 à 4, **caractérisée en ce que** le polymère séquencé comprend au moins une première séquence et au moins une deuxième séquence, la première séquence ayant une température de transition vitreuse (Tg) supérieure ou égale à 40°C et la deuxième séquence ayant une température de transition vitreuse inférieure ou égale à 20°C, lesdites première et deuxième séquences étant reliées entre elles par un segment intermédiaire comprenant au moins un monomère constitutif de la première séquence et au moins un monomère constitutif de la deuxième séquence.

**18.** Composition selon la revendication précédente, **caractérisée en ce que** la première séquence du polymère séquencé est issue en totalité ou en partie de un ou plusieurs monomères, qui sont tels que l'homopolymère préparé à partir de ces monomères a une température de transition vitreuse supérieure ou égale à 40°C.

**19.** Composition selon la revendication 17 **caractérisée en ce que** la première séquence est un copolymère issu de monomères qui sont tels que l'homopolymère préparé à partir de ces monomères a une température de transition vitreuse supérieure ou égale à 40°C.

**20.** Composition selon la revendication 18 ou 19 **caractérisée en ce que** les monomères dont l'homopolymère correspondant a une température de transition vitreuse supérieure ou égale à 40°C sont choisis parmi les monomères suivants :

- les méthacrylates de formule $CH_2$ = $C(CH_3)$-$COOR_1$ dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$

à $C_{12}$,

- les acrylates de formule $CH_2$ = CH-COOR$_2$
  dans laquelle $R_2$ représente un groupe cycloalkyle en $C_4$ à $C_{12}$ tel que l'acrylate d'isobornyle ou un groupe tertio butyle,
- les (méth)acrylamides de formule :

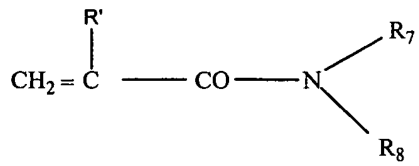

  où $R_7$ et $R_8$ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl, et R' désigne H ou méthyle,
- et leurs mélanges.

**21.** Composition selon l'une des revendications 18 à 20 **caractérisée en ce que** les monomères dont l'homopolymère correspondant a une température de transition vitreuse supérieure ou égale à 40°C sont choisis parmi le méthacrylate de méthyle, le méthacrylate d'isobutyle, le (méth)acrylate d'isobornyle et leurs mélanges.

**22.** Composition selon l'une des revendications 17 à 21 **caractérisée en ce que** la proportion de la première séquence du polymère séquencé va de 20 à 90% en poids du polymère, mieux de 30 à 80% et encore mieux de 50 à 70%.

**23.** Composition selon l'une des revendications 17 à 22 **caractérisée en ce que** la deuxième séquence du polymère séquencé est issue en totalité ou en partie de un ou plusieurs monomères, qui sont tel(s) que l'homopolymère préparé à partir de ces monomères a une température de transition vitreuse inférieure ou égale à 20°C.

**24.** Composition selon l'une des revendications 17 à 22 **caractérisée en ce que** la deuxième séquence est un homopolymère issu de monomères qui sont tel(s) que l'homopolymère préparé à partir de ces monomères a une température de transition vitreuse inférieure ou égale à 20°C.

**25.** Composition selon l'une des revendications 23 ou 24 **caractérisée en ce que** les monomères dont l'homopolymère correspondant a une température de transition vitreuse inférieure ou égale à 20°C sont choisis parmi les monomères suivants :

- les acrylates de formule $CH_2$ = CHCOOR$_3$,
  $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S ;
- les méthacrylates de formule $CH_2$ = C($CH_3$)-COOR$_4$,
  $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule $R_5$-CO-O-CH = $CH_2$
  où $R_5$ représente un groupe alkyle en $C_4$ à $C_{12}$ linéaire ou ramifié ;
- les éthers d'alcool vinylique et d'alcool en $C_4$ à $C_{12}$, ;
- les N-alkyl en $C_4$ à $C_{12}$ acrylamides, tels que le N-octylacrylamide,
- et leurs mélanges.

**26.** Composition selon l'une des revendications 23 à 25 **caractérisée en ce que** les monomères dont l'homopolymère correspondant a une température de transition vitreuse inférieure ou égale à 20°C sont choisis parmi les acrylates d'alkyle dont la chaîne alkyle comprend de 1 à 10 atomes de carbone, à l'exception du groupe tertiobutyle.

**27.** Composition selon l'une des revendications 20 à 26 **caractérisée en ce que** la proportion de la deuxième séquence ayant une Tg inférieure ou égale à 20°C du polymère séquencé va de 5 à 75% en poids du polymère, mieux de

15 à 50% et encore mieux de 25 à 45%.

**28.** Composition selon l'une des revendications 1 à 4, **caractérisée en ce que** le polymère séqueuncé comprend au moins une première séquence et au moins une deuxième séquence, la première séquence ayant une température de transition vitreuse (Tg) comprise entre 20 et 40°C et la deuxième séquence ayant une température de transition vitreuse inférieure ou égale à 20°C ou une température de transition vitreuse supérieure ou égale à 40°C, lesdites première et deuxième séquences étant reliées entre elles par un segment intermédiaire comprenant au moins un monomère constitutif de la première séquence et au moins un monomère constitutif de la deuxième séquence.

**29.** Composition selon la revendication précédente, **caractérisée en ce que** la première séquence ayant une Tg comprise entre 20 et 40°C du polymère séquencé est issue en totalité ou en partie de un ou de plusieurs monomères, qui sont tel(s) que l'homopolymère préparé à partir de ces monomères a une température de transition vitreuse comprise entre 20 et 40°C.

**30.** Composition selon la revendication 28 ou 29, **caractérisée en ce que** la première séquence ayant une Tg comprise entre 20 et 40°C du polymère séquencé est un copolymère issu de monomères qui sont tel(s) que l'homopolymère correspondant a une Tg supérieure ou égale à 40°C et de monomères qui sont tel(s) que l'homopolymère correspondant a une Tg inférieure ou égale à 20°C.

**31.** Composition selon l'une des revendications 28 à 30, **caractérisée en ce que** la première séquence ayant une Tg comprise entre 20 et 40°C du polymère séquencé est issue de monomères choisis parmi le méthacrylate de méthyle, l'acrylate et le méthacrylate d'isobornyle, l'acrylate de butyle, l'acrylate d'éthyl-2 hexyle et leurs mélanges.

**32.** Composition selon l'une des revendications 28 à 31, **caractérisée en ce que** la proportion de la première séquence ayant une Tg comprise entre 20 et 40°C du polymère séquencé va de 10 à 85% en poids du polymère, mieux de 30 à 80% e t encore mieux de 50 à 70%.

**33.** Composition selon l'une des revendications 28 à 32, **caractérisée en ce que** la deuxième séquence du polymère séquencé a une Tg supérieure ou égale à 40°C et est issue en totalité ou e n partie de un ou plusieurs monomères, q ui sont tels q ue l'homopolymère préparé à partir de ces monomères a une température de transition vitreuse supérieure ou égale à 40°C.

**34.** Composition selon l'une des revendications 28 à 33, **caractérisée en ce que** la deuxième séquence du polymère séquencé a une Tg supérieure ou égale à 40°C et est un homopolymère issu de monomères, qui sont tels que l'homopolymère préparé à partir de ces monomères a une température de transition vitreuse supérieure ou égale à 40°C.

**35.** Composition selon l'une des revendications 30 à 34, **caractérisée en ce que** les monomères dont l'homopolymère correspondant a une température de transition vitreuse supérieure ou égale à 40°C sont choisis parmi les monomères suivants :

- les méthacrylates de formule $CH_2 = C(CH_3)\text{-}COOR_1$
  dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,
- les acrylates de formule $CH_2 = CH\text{-}COOR_2$
  dans laquelle $R_2$ représente un groupe cycloalkyle en $C_4$ à $C_{12}$ tel que l'acrylate d'isobornyle ou un groupe tertio butyle,
- les (méth)acrylamides de formule :

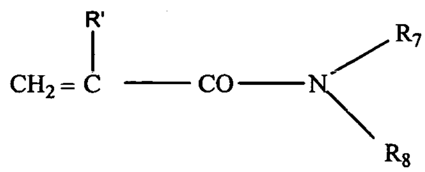

où $R_7$ et $R_8$ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl, et R' désigne H ou méthyle,

- et leurs mélanges.

**36.** Composition selon l'une des revendications 30 à 35, **caractérisée en ce que** les monomères dont l'homopolymère correspondant a une température de transition vitreuse supérieure ou égale à 40°C sont choisis parmi le méthacrylate de méthyle, le méthacrylate d'isobutyle, le (méth)acrylate d'isobornyle et leurs mélanges.

**37.** Composition selon l'une des revendications 28 à 36, **caractérisée en ce que** la proportion de la deuxième séquence ayant une Tg supérieure ou égale à 40°C du polymère séquencé va de 10 à 85%, de préférence de 20 à 70% et mieux de 30 à 70% en poids du polymère.

**38.** Composition selon l'une des revendications 28 à 32, **caractérisée en ce que** la deuxième séquence a une Tg inférieure ou égale à 20°C et est issue en totalité ou en partie de un ou plusieurs monomères, qui sont tel(s) que l'homopolymère préparé à partir de ces monomères a une température de transition vitreuse inférieure ou égale à 20°C.

**39.** Composition selon l'une des revendications 28 à 32, **caractérisée en ce que** la deuxième séquence du polymère séquencé a une Tg inférieure ou égale à 20°C et est un homopolymère issu de monomères qui sont tel(s) que l'homopolymère préparé à partir de ces monomères a une température de transition vitreuse inférieure ou égale à 20°C.

**40.** Composition selon la revendication 38 ou 39, **caractérisée en ce que** les monomères dont l'homopolymère correspondant a une température de transition vitreuse inférieure ou égale à 20°C sont choisis parmi les monomères suivants :

- les acrylates de formule $CH_2 = CHCOOR_3$,
  $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S ;
- les méthacrylates de formule $CH_2 = C(CH_3)\text{-}COOR_4$,
  $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule $R_5\text{-CO-O-CH} = CH_2$
  où $R_5$ représente un groupe alkyle en $C_4$ à $C_{12}$ linéaire ou ramifié ;
- les éthers de vinyle et d'alkyle en $C_4$ à $C_{12}$, tels que l'éther de vinyle et de méthyle et l'éther de vinyle et d'éthyl ;
- les N-alkyl en $C_4$ à $C_{12}$ acrylamides, tels que le N-octylacrylamide
- et leurs mélanges.

**41.** Composition selon l'une des revendications 38 à 40, **caractérisée en ce que** les monomères dont les homopolymères ont des températures de transition vitreuse inférieures ou égales à 20°C sont choisis parmi les acrylates d'alkyle dont la chaîne alkyle comprend de 1 à 10 atomes de carbone, à l'exception du groupe tertiobutyle.

**42.** Composition selon l'une des revendications 38 à 41, **caractérisée en ce que** la proportion de la séquence ayant une température de transition vitreuse inférieure ou égale à 20°C du polymère séquencé va de 20 à 90% en poids du polymère, mieux de 30 à 80% et encore mieux de 50 à 70%.

**43.** Composition selon l'une des revendications précédentes, **caractérisée en ce que** la première séquence et/ou la deuxième séquence du polymère séquencé comprend au moins un monomère additionnel.

**44.** Composition selon la revendication précédente, **caractérisée en ce que** le monomère additionnel est choisi parmi les monomères hydrophiles, les monomères à insaturation éthylénique comprenant un ou plusieurs atomes de silicium et leurs mélanges.

**45.** Composition selon la revendication 43 ou 44, **caractérisée en ce que** le monomère additionnel est choisi parmi :

- les monomères à insaturation(s) éthylénique(s) comprenant au moins une fonction acide carboxylique ou sulfonique
- les méthacrylates de formule $CH_2 = C(CH_3)\text{-}COOR_6$
  dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,
- les méthacrylates de formule $CH_2 = C(CH_3)\text{-}COOR_9$,
  $R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;
- les acrylates de formule $CH_2 = CHCOOR_{10}$,
  $R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle($C_1$-$C_{12}$)-O-POE (polyoxyéthylène) avec répétition du motif oxyéthylène de 5 à 30 fois, par exemple méthoxy-POE, ou $R_{10}$ représente un groupement polyoxyéthylèné comprenant de 5 à 30 motifs d'oxyde d'éthylène
- les monomères à insaturation(s) éthylénique(s) comprenant au moins une fonction amine tertiaire, et leurs mélanges.

**46.** Composition selon l'une des revendications 43 à 45, **caractérisée en ce que** le ou les monomères additionnels sont choisis parmi l'acide acrylique, l'acide méthacrylique, le méthacrylate de trifluoroéthyle et leurs mélanges.

**47.** Composition selon l'une des revendications 43 à 46, **caractérisée en ce que** le ou les monomères additionnel(s) représente(nt) de 1 à 30% en poids du poids total des première et/ou deuxième séquences du polymère séquencé.

**48.** Composition selon l'une des revendications précédentes, **caractérisé en ce que** chacune des première et deuxième séquence du polymère séquencé comprend au moins un monomère choisi parmi les esters d'acide (meth) acrylique, et éventuellement au moins un monomère choisi parmi l'acide (meth)acrylique et leurs mélanges.

**49.** Composition selon l'une des revendications précédentes, **caractérisé en ce que** chacune des première et deuxième séquence du polymère séquencé est issue en totalité d'au moins un monomère choisi parmi l'acide acrylique, les esters d'acide (méth)acrylique, et éventuellement d'au moins un monomère choisi parmi l'acide (méth)acrylique, et leurs mélanges.

**50.** Composition selon l'une des revendications précédentes, **caractérisée en ce que** l'écart entre les températures de transition vitreuse (Tg) des première et deuxième séquences du polymère séquencé est supérieur à 10°C, mieux, supérieur à 20°C, de préférence supérieure à 30°C et mieux supérieure à 40°C.

**51.** Composition selon l'une des revendications précédentes, **caractérisée en ce que** la séquence intermédiaire du polymère séquencé a une température de transition vitreuse comprise entre les températures de transition vitreuse des première et deuxième séquences.

**52.** Composition selon l'une des revendications précédentes, **caractérisée en ce que** le polymère séquencé a un indice de polydispersité I supérieur à 2, de préférence supérieur ou égal à 2,5, de préférence supérieure ou égal à 2,8.

**53.** Composition selon l'une des revendications précédentes, **caractérisée en ce que** le polymère séquencé a un indice de polydispersité compris entre 2,8 et 6.

**54.** Composition selon l'une des revendications précédentes, **caractérisée en ce que** le polymère séquencé est un polymère éthylénique séquencé linéaire filmogène.

**55.** Composition selon l'une des revendications précédentes, **caractérisée en ce que** le polymère séquencé a une masse moyenne en poids (Mw) inférieure ou égale à 300 000.

**56.** Composition selon l'une des revendications précédentes, **caractérisée en ce que** le polymère séquencé a une masse moyenne en poids (Mw) allant de 35 000 à 200 000, et mieux de 45 000 à 150 000.

**57.** Composition selon l'une des revendications précédentes, **caractérisée en ce que** le polymère séquencé a une masse moyenne en nombre (Mn) inférieure ou égale à 70 000.

**58.** Composition selon l'une des revendications précédentes, **caractérisée en ce que** le polymère séquencé a une masse moyenne en nombre (Mn) allant de 10 000 à 60 000, et mieux de 12 000 à 50 000.

**59.** Composition selon l'une des revendications précédentes, **caractérisée en ce que** le polymère séquencé n'est pas soluble à une teneur en matière active d'au moins 1% en poids dans l'eau ou dans un mélange d'eau et de monoalcools inférieurs linéaires ou ramifiés ayant de 2 à 5 atomes de carbone, sans modification de pH, à température ambiante (25°C).

**60.** Composition selon l'une des revendications précédentes, **caractérisée en ce que** le polymère séquencé n'est pas un élastomère.

**61.** Composition selon l'une des revendications précédentes, **caractérisée en ce que** le polymère séquencé représente de 0,1 à 60 en poids par rapport au poids total de la composition, de préférence de 0,5 à 50% et mieux de 1 à 40% en poids.

**62.** Composition selon l'une des revendications précédentes, **caractérisée en ce que** le milieu solvant organique comprend un solvant organique choisi parmi :

- les cétones liquides à température ambiante tels que les méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la γ-butyrolactone ;
- les esters à chaîne courte (ayant de 3 à 8 atomes de carbone au total) tels que l'acétate d'éthyle, l'acétate de méthyle, l'acétate de propyle, l'acétate d'isopropyl, l'acétate de n-butyle, l'acétate d'isopentyle, l'acétate de méthoxypropyle, le lactate de butyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde ;
- les composés hétérocycliques tels que le tétrahydrofuranne ;
- le carbonate de propylène, le 3-éthoxypropionate d'éthyle ;
- leurs mélanges.

**63.** Composition selon l'une des revendications précédentes **caractérisée en ce que** le milieu solvant organique présente une polarité P allant de 0,422 à 0,725.

**64.** Composition selon l'une des revendications précédentes, **caractérisée en ce que** le milieu solvant organique représente de 10 à 95% en poids, par rapport au poids total de la composition, de préférence de 15% à 80% en poids, et mieux de 20 à 60% en poids.

**65.** Composition selon l'une des revendications précédentes, **caractérisée en ce qu'**elle comprend un polymère filmogène additionnel.

**66.** Composition selon la revendication précédente, **caractérisée** en en ce que le polymère filmogène est présente en une teneur allant de 0,1 % à 60 % en poids, par rapport au poids total de la composition, de préférence allant de 2 % à 40 % en poids, et mieux de 5 % à 25 % en poids.

**67.** Composition selon l'une des revendications précédentes, **caractérisée en ce qu'**elle comprend un agent plastifiant en une quantité inférieure à 20%, de préférence inférieur à 15%, mieux inférieure à 10% et encore mieux inférieure à 5% en poids par rapport au poids total de la composition.

**68.** Composition selon l'une des revendications précédentes, **caractérisée en ce qu'**elle comprend une matière colorante.

**69.** Composition selon la revendication précédente, **caractérisée en ce que** la matière colorante est présent en une teneur allant de 0,01 % à 50 % en poids, par rapport au poids de la composition, de préférence de 0,01 % à 30 % en poids.

**70.** Procédé cosmétique de maquillage ou de soin non thérapeutique des ongles comprenant l'application sur les ongles d'au moins une couche d'une composition de vernis à ongles selon l'une des revendications 1 à 69.

**71.** Utilisation d'une composition de vernis à ongles comprenant au moins un polymère séquencé selon l'une quelconque des revendications 1 à 69, ladite composition étant apte à former un film présentant un pouvoir amortissant tangente delta (tgδ) supérieur ou égal à 0,4 à une température de 30 °C et une fréquence de 20 Hz, pour obtenir un film, déposé sur les ongles, brillant, de bonne tenue et résistant à l'usure.

**72.** Ensemble cosmétique comprenant : i) un récipient délimitant au moins un compartiment, ledit récipient étant fermé par un élément de fermeture et ii) une composition disposée à l'intérieur dudit compartiment, la composition étant conforme à l'une quelconque des revendications 1 à 69.

**73.** Ensemble cosmétique selon la revendication 72 **caractérisé en ce que** le récipient est formé, au moins pour partie, en verre.

**74.** Ensemble cosmétique selon la revendication 72 **caractérisé en ce que** le récipient est formé, au moins pour partie, en au moins un matériau autre que le verre, par exemple un matériau thermoplastique ou un métal.

**75.** Ensemble selon l'une quelconque des revendications 72 à 74 **caractérisé en ce que**, en position fermée du récipient, l'élément de fermeture est vissé sur le récipient.

**76.** Ensemble selon l'une quelconque des revendications 72 à 74 **caractérisé en ce que**, en position fermée du récipient, l'élément de fermeture est couplé au récipient autrement que par vissage, notamment par encliquetage.

**77.** Ensemble selon l'une quelconque des revendications 72 à 76 **caractérisé en ce qu'**il comporte un applicateur sous forme d'un pinceau comprenant au moins une touffe de poils.

**78.** Ensemble selon l'une quelconque des revendications 72 à 76 **caractérisé en ce qu'**il comporte un applicateur different d'un pinceau, tel qu'une spatule.